# EUROPÄISCHE PATENTSCHRIFT

(11) **EP 0 708 111 B1**
(45) Veröffentlichungstag und Bekanntmachung des Hinweises auf die Patenterteilung: **05.01.2000**
(21) Anmeldenummer: 95114511.9
(22) Anmeldetag: 15.09.1995
(51) Int. Cl.: C07J 5/00, A61K 31/57, C07J 43/00, C07J 41/00, C07J 17/00, C07J 33/00, C07J 31/00, A61K 31/58

(54) **17-Desoxi-corticosteroid-21-[O]-Carbonsäureester, Verfahren zu deren Herstellung und diese enthaltende Arzneimittel**
17-Desoxy corticosteroid-21-esters, a process for their preparation and pharmaceutical compositions containing them
21-Esters des 17-désoxycorticostéroides, un procédé de leur production et les médicaments les contenant

(30) Priorität: 20.09.1994 DE 4433374
(43) Veröffentlichungstag der Anmeldung: 24.04.1996
(73) Patentinhaber: HOECHST AKTIENGESELLSCHAFT, 65929 Frankfurt am Main (DE)
(72) Erfinder: Stache, Ulrich, Dr., D-65719 Hofheim (DE); Alpermann, Hans-Georg, Dr., D-61462 Königstein (DE); Bohn, Manfred, Dr., D-65719 Hofheim (DE)

(56) Entgegenhaltungen:
- EP-A- 0 127 829
- CH-A- 495 969
- DE-B- 1 131 668
- US-A- 2 755 292
- US-A- 2 783 226
- US-A- 4 086 254
- JOURNAL OF MEDICINAL CHEMISTRY, Bd. 12, Nr. 5, September 1969 WASHINGTON US, Seiten 810-818, M. WALL ET AL 'The Effects of Some Steroidal Alkylating Agents on Experimental Animal Mammary Tumor and Leukemia Systems'
- COMPTES RENDUS HEBDOMADAIRES DES SEANCES DE L'ACADEMIE DES SCIENCES, Bd. 239, 1954 MONTREUIL FR, Seiten 14-16, R. COURRIER ET AL 'Sur les propriétés biologiques des allénolates de désoxycortiocostérone'
- CHEMICAL ABSTRACTS, vol. 51, no. 13, 1957 Columbus, Ohio, US; abstract no. 9723h, A. ERCOLI 'Steroids with side chain containing a ketol group' Spalte 9723; & ES-A-229 413 (FRANCISCO VISMARA SOCIET PER AZIONI & DROGAS VACUNAS Y SUEROS) 31.Juli 1956
- CHEMICAL ABSTRACTS, vol. 112, no. 19, 1990 Columbus, Ohio, US; abstract no. 178253, H. FUJINO ET AL 'Synthesis and reactivity of anthracene-1-carbonyl azide as a fluorescent derivatization reagent for alcohols' Seite 696; Spalte 1; & YAGUGAKU ZASSHI, Bd. 109, Nr. 8, 1989 Seiten 606-610, & 'Chemical Abstracts Twelfth Collective Index, Volumes 106-115, Formulas C32H57BO5-C40H14I2N4O2' 1991 , CHEMICAL ABSTRACTS , COLUMBUS OHIO, US
- IL FARMACO, Edizione Scientifica, Band 30, Seiten 167-184 (1975) G. CARENINI et al., Laboratori Ricerche Della Maggioni & C.S.P.A., Milano, IT

## Beschreibung

17-Desoxi-corticosteroid-21-[O]-Carbonsäureester, Verfahren zu deren Herstellung und diese enthaltende Arzneimittel

Die Erfindung betrifft 17-Desoxi-corticoid-21-Carbonsäureester der Formel I 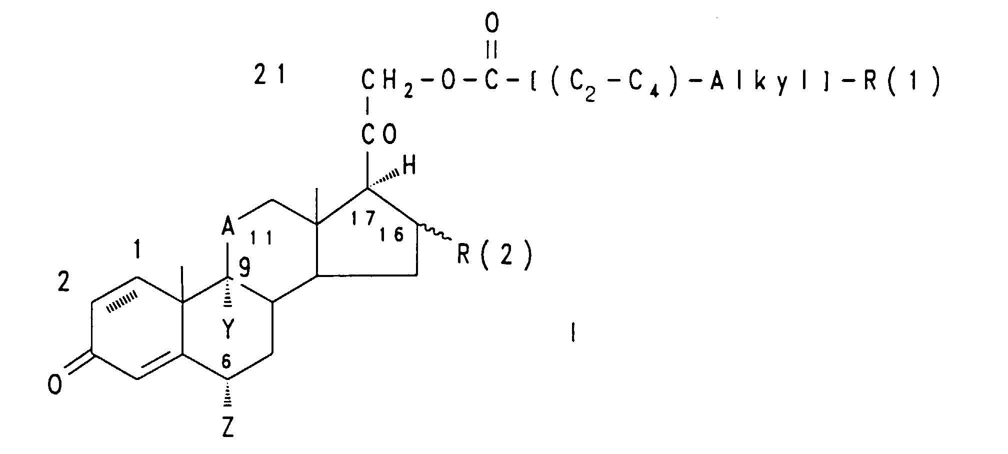 in welcher bedeuten:
- A: CHOH und CHCI in beliebiger sterischer Anordnung, CH₂ oder C=O;
- Y: Wasserstoff, Fluor oder Chlor;
- Z: Wasserstoff, Fluor oder Methyl;
- R(1): ggf. substituiertes oder annelliertes Aryl, Heteroaryl;
- [(C₂-C₄)-Alkyl]: ungesättigt, ab C₃ auch mehrfach ungesättigt;
die Positionen 1,2 gesättigt oder ungesättigt sind,
- R(2): Wasserstoff, α- oder β-Methyl.

Bevorzugt sind 17-Desoxi-corticoid-21-carbonsäureester der Formel I, in denen bedeuten:
- R(1): wie in Anspruch 1 definiert;
- A: CHOH, das β-konfiguriert ist;
- Y: F;
- Z: Wasserstoff;
- R(2): α-Methyl.

Die Erfindung betrifft auch ein Verfahren zum Herstellen einer Verbindung I, bei welchem man
a) eine Verbindung der Formel II, 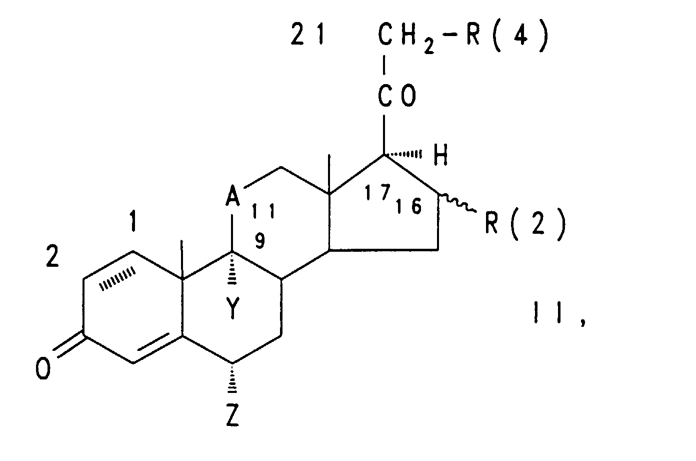 in der R(4) gleich OH ist und die übrigen Substituenten die oben angegebenen Bedeutungen haben,
   a 1) mit einer aktivierten Carbonsäure der Formel III, vorzugsweise einem Halogenid oder Anhydrid oder Azolid,

      R(5)-CO-[(C₂-C₄)-Alkyl]-R(1) III

      umsetzt, wobei
      [(C₂-C₄)-Alkyl] und R(1) die in Anspruch 1 angegebenen Bedeutungen haben und
      - R(5): Cl, Br, O[-CO-[(C₂-C₄)-Alkyl]-R(1)]₁-, -OC(O)CF₃ oder ein anderes aktiviertes Säureradikal, oder
   a 2) mit einer Carbonsäure der Formel III selbst, in der
      - R(5): OH
      und die weiteren Substituenten bei Formel III angegeben sind,
      in Gegenwart Wasser abspaltender Reagentien umsetzt
      oder daß man
b) Verbindungen der Formel II, 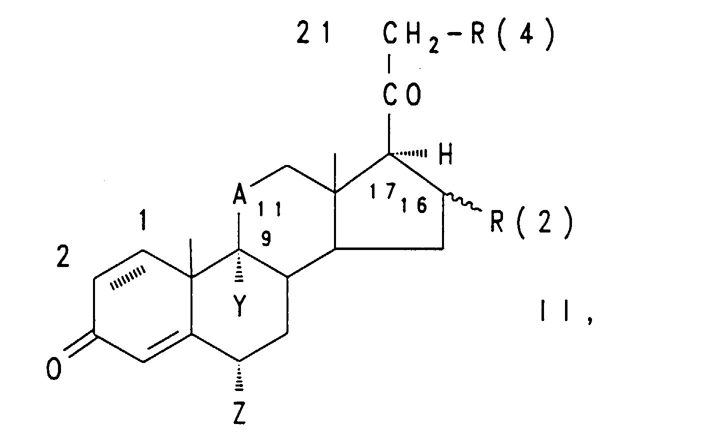 in der R(4) = Br, J, eine Sulfonsäurearyl- oder -alkylestergruppierung ist und die weiteren Substituenten die bei Formel I angegebenen Bedeutung haben, mit einem Salz, vorzugsweise K- oder Na-Salz oder einem Trialkylammoniumsalz, einer Carbonsäure der Formel III,

   R(5)-CO-[(C₂-C₄)-Alkyl]-R(1) III

   in der
   - R(5): -[O⁻Me⁺]
   und die weiteren Substituenten die bei Formel III angegebenen Bedeutungen haben,
   umsetzt,
   wobei Me⁺ vorzugsweise das Kation eines Alkalisalzes oder eines Trialkylammoniumsalzes ist.

Die punktierte Linie zwischen den C-Atomen 1 und 2 zeigt an, daß diese Bindung eine Einfachbindung oder eine ungesättigte Bindung sein kann.

Als Aryl- und Hetarylgruppen kommen bevorzugt infrage: Phenyl, Naphthyl, Biphenylyl, Phenyloxy, Phenylthio, Benzoyl, Thienyl, Furyl, Thiazolyl, Pyrrolyl, Imidazolyl, Pyridyl, Indolyl, Xanton-oxy, Flavonyl. Diese Aryl- und Heteroarylgruppen sind unsubstituiert oder substituiert mit 1 - 3 Substituenten ausgewählt aus der Gruppe bestehend aus (C₁-C₁₂)-Alkyl (gesättigt oder ungesättigt), F, Cl, Br, I, (C₁-C₈)-Alkoxy (gesättigt oder ungesättigt; zwei benachbarte Gruppen können auch Methylendioxy-Gruppen bilden), NO₂, (C₁-C₄)-Alkylthio, Phenoxy, Benzoyl, NR(6)R(7) mit R(6) und R(7) gleich oder verschieden gleich Wasserstoff, (C₁-C₄)-Alkyl, (C₁-C₄)-Acyl, t-Butyl-oxycarbonyl, (CH₂)-CH₂Cl; außerdem können die aromatischen Ringe in den Substituenten auf den Aryl- und Heteroarylgruppen ihrerseits unsubstituiert sein oder substituiert mit 1 - 3 Substituenten ausgewählt aus der Gruppe bestehend aus (C₁-C₄)-Alkyl, (C₁-C₄)-Alkoxy, F, Cl, Br und 1. Die als Ausgangssubstanzen benötigten 17-Desoxi-steroide mit freier 21-Hydroxylgruppe der Formel II [R(4) = OH] sind in der Regel literaturbekannt.

Die 17-Desoxi-steroide mit R(4) gleich Br, J, -OSO₂-Aryl, -OSO₂-Alkyl in Formel II werden in Analogie zu der US-Patentschrift 4 377 575 (HOE 78/F 082) hergestellt. Hierbei kommen beispielsweise folgende 1 7-Desoxi-corticosteroide in Frage:
Corticosteron (11β,21-Dihydroxypregn-4-on-3,20-dion)
Desoxycorticosteron (11-Deoxycorticosteron)
16α-Methyl-1(2)dehydro-corticosteron
6α-Fluor-16α-methyl-1(2)-dehydro-corticosteron (= Fluocortolon)
9α-Fluor-16α-methyl-1(2)-dehydro-corticosteron (= Desoximetason)
Diflucortolon
Clocortolon
16α-Methyl-1(2),9(11)-di-dehydro-corticosteron
6α,9α-Difluor-corticosteron
9α-Fluor-corticosteron
6α-Methyl-corticosteron
6α-Fluor-corticosteron
11α-Hydroxy-1(2)-dehydro-11-desoxy-corticosteron
6α,16α-Dimethyl-corticosteron
11-Dehydro-desoximetason

Die als Reaktionspartner zum Einsatz kommenden Carbonsäuren der Formel III R(5) gleich OH bzw. deren aktivierte Derivate, wie die Halogenide R(5) = Cl, Br, J oder deren Anhydride, oder deren Azolide R(5) gleich Imidazolid, Triazolid oder deren Salze [R(5) gleich [Me⁺O⁻]-, vorzugsweise [K⁺O⁻]-, [Na⁺O⁻]-] sind in der Regel bekannt und werden ggf. nach allgemeinen präparativen Methoden hergestellt. Beispiele der gemäß der Erfindung zum Einsatz gelangenden Carbonsäuren gemäß Formel III R(5) gleich OH findet man in der Liste am Ende des Textes vor den Ansprüchen.

Alle hierunter fallenden Carbonsäuren tragen in ihrem Säurerest eine ggf. durch Halogen, Alkyl, Alkoxyl, Acyl, Thioalkyl- oder -acyl, Nitro, Amin, Aminalkyl, Amido, Cyan, Oxyacyl, Oxyaryl etc. substituierte oder auch ggf. annellierte Aryl- oder Hetarylgruppe. Letztere sind essentieller Bestandteil der Erfindung.

Wie im pharmakologischen Teil gezeigt wird, zeigen insbesondere 17-Desoxicorticoid-21-carbonsäureester dieses Typs ( = 21-Aryl- bzw. -Hetarylester-Typ) im Vergleich zu strukturverwandten Corticoid-21-carbonsäureestern, die keine Aryl- bzw. Heteroarylgruppe im 21-Säurerest tragen bzw. im Vergleich zu 17-Desoxi-corticoiden mit nicht veresterter freier 21-Hydroxlgruppe oft deutlich bessere Wirkqualitäten hinsichtlich des Verhältnisses lokale/systemische antiinflammatorische Wirkung.

Detaillierte Beschreibung der einzelnen Reaktionsführungen der Herstellungsverfahren für die erfindungsgemäßen Verfahrensprodukte gemäß Formel I:

### zu Verfahrensvariante a:

Zur Herstellung von 21-Carbonsäureestern des o. a. Typs werden vorzugsweise entweder Carbonsäurehalogenide oder -azolide der Formel IV

R(5)-OC-[(C₂-C₄-Alkyl]-R(1) IV,

in der bedeuten:
- R(5): Cl, Br, J, 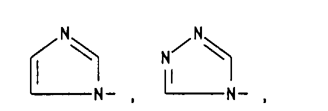
und
R(1) sowie (C₂-C₄)-Alkyl die zur Formel III angegebenen Bedeutungen haben
oder Carbonsäureanhydride der Formel V

O{-OC-[(C₂-C₄)-Alkyl]-R(1)}₂ V,

in der bedeuten:

R(1) sowie (C₂-C₄)-Alkyl die zur Formel III angegebenen Bedeutungen haben, verwendet. In beiden Fällen können die ihnen zugrundeliegenden in der Liste aufgeführten Carbonsäuren verwendet werden, vorzugsweise deren Carbonsäurechloride, -anhydride und -imidazolide bzw. -triazolide.

R(5) in Formel IV können auch andere die Carboxylgruppe in Carbonsäuren die Veresterung aktivierende Gruppen beinhalten, so beispielsweise -O-CO-CF₃ oder die aus Phosphon- oder Phosphinsäureanhydriden (z.B. Propanphosphonsäureanhydrid) oder Polyphosphorsäureanhydrid (PPA) herstellbaren aktivierten Carbonsäuren.

Weitere Phosphorreagentien, die eine schonende Veresterung von organischen Carbonsäuren mit der 21-Alkoholgruppe von Corticoid-17-alkylcarbonaten bewirken können sind in den Literaturstellen Synth. Commun. 13, 471ff (1983) und Synth. Commun. 14, 515ff (1984) angeführt bzw. beschrieben.

Zur Veresterung mit einem Carbonsäurehalogenid oder -anhydrid löst man die Steroidkomponente in einem inerten Lösungsmittel, beispielsweise in einem Ether, wie Dioxan, Tetrahydrofuran, Diglym, oder gegebenenfalls halogenierten Kohlenwasserstoffen, wie Benzol, Toluol, Cyclohexan, Methylenchlorid, Chloroform oder in Aceton oder in einem Gemisch dieser Lösungsmittel. Zur Entfernung der in der Reaktion entstehenden Halogenwasserstoffsäure setzt man 1 bis 1000 Moläquivalente einer tertiären Base, wie Pyridin, Chinolin, Triethylamin, Dimethylanilin, Dimethylaminopyridin usw., zu. Man kann aber auch eine anorganische Base, wie Natriumhydrogencarbonat oder Calciumcarbonat, zur Entfernung der Säure benutzen. Anschließend tropft man 1 bis 200 Moläquivalente, vorzugsweise 1 bis 3 Moläquivalente eines der oben angeführten Acylierungsmittel, gegebenenfalls gelöst in einem der oben angeführten Lösungsmittel, bei einer Temperatur von -40°C bis zum Siedepunkt des verwendeten Lösungsmittels, vorzugsweise von O bis 25°C, zu. Anschließend läßt man das Reaktionsgemisch eine bis 120 Stunden bei einer Temperatur von -40°C bis zum Siedepunkt des Lösungsmittels, vorzugsweise von O bis 25°C stehen.

Bei Verwendung von Carbonsäureanhydriden als Acylierungsmittel ist es hin und wieder von Vorteil, ohne Zusatz von Lösungsmitteln zu arbeiten. Es reicht in der Regel aus, lediglich die organische Base, vorzugsweise Pyridin, dem gegebenenfalls im Überschuß angewandten Säureanhydrid zuzufügen.

Insbesondere bei empfindlichen (und zuweilen instabilen) Carbonsäurederivaten des o. a. Typs, insbesondere bei Verwendung von Phenylacetylchloriden, -anhydriden, Hetarylacetylchloriden und -anhydriden, ist es von großem präparativem und reaktionsselektivem Vorteil, die 17-Desoxi-corticoide mit freier 21-Hydroxylgruppe mit 1 bis 4 Moläquivalenten des Chlorids bzw. Anhydrids bei -10 bis +6° (maximal 20°C) in chlorierten Kohlenwasserstoffen, wie vorzugsweise Dichlormethan, sowie mit 1 bis 4 Moläquivalenten einer Pyridinbase, vorzugsweise Dimethylaminopyridin, umzusetzen.

Hierbei werden die Reaktionsprodukte der Formel I in hoher Reinheit, ohne nennenswerte Beimengungen an Nebenprodukten, insbesondere 11-acylierten Produkten, erhalten (Verfolgung der Reaktionsführungen durch DC), das heißt die Reaktionsführungen sind hinsichtlich der Umsetzung der 21-Hydroxygruppe hoch regioselektiv.

Bei den Reaktionen mit Carbonsäurechloriden wird in vorteilhafter Weise oft absolutes Dioxan oder Tetrahydrofuran zum Reaktionsgemisch gegeben, z.B. bei Benzoylchlorid, wobei das Verhältnis Dioxan/Pyridin etwa 1 : 1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).

Die Charakterisierung der Reaktionsprodukte kann durch Dünnschicht-Chromatographie (DC) erfolgen; hierbei haben die Reaktionsprodukte R_{F}-Werte von etwa 0.6 bis 0.8. In der Regel werden die Reaktionsprodukte durch Massenspektren mit MS = m/z = .... (M + H⁺) charakterisiert (in der Regel FAB-Spektren); es werden jeweils die monoisotopischen Molmassen erfaßt. Die M + H⁺-Werte wurden jeweils aufgerundet. Auch IR-, ¹H-NMR- und UV-Spektren können zur Charakterisierung herangezogen werden.

Zur Aufarbeitung gießt man das Reaktionsgemisch in Wasser, das gegebenenfalls mit Natriumchlorid und Natriumbicarbonat versetzt wurde, wobei die Reaktionsprodukte, oft erst nach längerem Stehen, im allgemeinen kristallin ausfallen. Ölig oder wachsartig gebliebene Reaktionsprodukte werden durch Ausschütteln mit einem geeigneten Extraktionsmittel und Eindampfen angereichert. Die Reaktionsprodukte können, falls erforderlich, durch Umkristallisieren oder durch Chromatographie aufgetrennt oder gereinigt werden. Oft genügt auch intensives Digerieren in einem das Reaktionsprodukt möglichst wenig oder nicht lösenden organischen Lösungsmittel, wie Diethylether oder Cyclohexan, oder einem Gemisch aus diesen Komponenten zur weiteren Reinigung der Reaktionsprodukte.

Bei Verwendung von Carbonsäureazoliden führt man die Veresterung zweckmäßig als Eintopfreaktion durch. Hierbei löst man beispielsweise Aryl- oder Heteroarylessigsäure oder eine andere Carbonsäure der Formel III [R(5) gleich OH], in absolutem Pyridin und gibt eine vorzugsweise äquimolare Menge N,N-Carbonyl-diimidazol oder -[1H-1,2,4-triazol] hinzu, wobei sich bei O bis 20° die entsprechenden Säureazolide bilden. Nach Zugabe einer etwa äquimolaren Menge Corticoid-17-alkylcarbonat der Formel II [R(5) = OH] und katalytischer Menge einer Base, vorzugsweise Natriumhydrid oder-imidazolid rührt man in Pyridin zwischen 0 bis 40°C; vorzugsweise 20° und arbeitet wie üblich auf.

Man kann aber auch das vorher durch äquimolare Mengen N,N'-Carbonylazolid und Carbonsäure in absolutem Tetrahydrofuran hergestellte und isolierte Carbonsäureazolid in Lösungsmitteln wie Pyridin, Dimethylformamid, Tetrahydrofuran im gelösten Steroid zugeben und weiter wie oben geschildert verfahren [s. auch Chem. Ber. 95, S. 1284 ff. (1962)].

Bei der Veresterung mit Hilfe von Phosphon- bzw. Phosphinsäureanhydriden setzt man vorzugsweise äquimolare Mengen Carbonsäure und Corticoid-21-alkohol in absolutem Pyridin mit 50 %igen Propanphosphorsäureanhydrid in Methylenchlorid bei 20 bis 60° unter Zugabe von 4-Dimethylaminopyridin als Säurefänger hinzu und arbeitet wie üblich auf (in Eiswasser eingießen, mit Essigester extrahieren, mit 5 % KHSO₄ waschen, abdestillieren, kristallisieren). Anstelle von Phosphonsäureanhydriden kann man auch Polyphosphorsäureanhydrid (PPA) einsetzen.

Ein weiteres vorteilhaftes Veresterungsverfahren, das auf die gemäß Formel III [R(5) gleich OH] oder in der Liste aufgeführten Carbonsäuren anwendbar ist, ist die direkte Umsetzung von 1 7-Desoxi-corticoide der Formel II [R(4) gleich OH] mit Hilfe von wasserentziehenden Mitteln, wie Carbodiimiden, vorzugsweise N,N'-Dicyclohexylcarbodiimid (DCCI). Anstelle von DCCi kann man in einigen Fällen auch mit "Molekularsieben" als wasserentziehenden Mitteln arbeiten.

Durch Zusatz einer Säure, z.B. Schwefelsäure, Phosphorsäure, Chlorwasserstoffsäure, Diphenylphosphorsäure, p-Toluolsulfonsäure bzw. von deren Pyridiniumsalzen oder einer organischen Base, wie z.B. Dimethylaminopyridin ( = besonders vorteilhaft in halogenierten Lösungsmitteln, wie z.B. Methylenchlorid, oder in Dimethyl-formamid) kann die Veresterung katalytisch beschleunigt bzw. optimiert werden, was insbesondere bei sonst schwer reagierenden bzw. empfindlichen Carbonsäuren, z.B. vom Indolylessigsäure-, Pyrrolcarbonsäure-, Aryl- und Hetarylessigsäuretyp usw. sehr vorteilhaft ist. Hierbei ist es überraschend, daß die sekundäre 11-Hydroxygruppe in den eingesetzten 1 7-Desoxi-corticoiden praktisch in der Regel nicht gleichzeitig mit verestert wird, wie man es öfter bei der Veresterung mit den entsprechenden Säurehalogeniden beobachtet.

In einer besonderen Verfahrensvariante gibt man zu einer Lösung von 1 Moläqu. 17-Desoxi-corticoid-21-alkohol [Formel II, R(4) gleich OH] und 1 bis 4 Moläqu. Carbonsäure der Formel III [R(5) gleich OH], vorzugsweise 2 Äquivalente, in absolutem Pyridin eine katalytische Menge Schwefelsäurepyridiniumsalz, sowie nach circa 20 Min. 1 bis 4 Moläqu. Dicyclohexylcarbodiimid, vorzugsweise 1 bis 2 Moläqu. zu. Man rührt hiernach bei 0 bis 50°C, vorzugsweise 20°C, bis eine DC-Probe keine Ausgangscarbonsäure, sondern nur gewünschte 17-Desoxi-corticoid-21-carbonsäureester der Formel I anzeigt. Man filtriert vom entstandenen Dicyclohexylharnstoff ab, gießt das Filtrat zweckmäßig in Wasser ein, filtriert (bei Kristallbildung) oder dekantiert (bei öligen bzw. wachsartigen Umfällungen) ab, wäscht mit Wasser nach (ggf. extrahiert man auch mit Extraktionsmittel, insbesondere Dichlormethan), trocknet, kristallisiert wie üblich um oder stellt, falls erforderlich, die Reaktionsprodukte durch übliche Chromatographie, vorzugsweise an Kieselgel, rein dar.

Anstelle von Pyridin können in einigen Fällen auch anderen inerte Lösungsmittel, wie z.B. Tetrahydrofuran, Dioxan, Methylenchlorid, Dimethylformamid zweckmäßig unter Hinzugabe von tertiären Basen, beispielsweise Pyridin, 4-Dimethylaminopyridin verwendet werden. Bei Verwendung von Molekularsieben als wasserentziehenden Mittel sind letztere Lösungsmittel vorzuziehen.

Für die Veresterung mit den empfindlichen Aryl- und Heteroarylessigsäuren hat sich weiterhin folgende Variante bewährt: 1 Äqu. Carbonsäure wird bei 0°C in absolutem Dichlormethan gelöst, und nacheinander wird mit 1 Äqu. DCCI, 0.2 Äqu. 4-N,N'-Dimethylaminopyridin und einer Lösung von 1 Äqu. 17-Desoxi-corticosteroid-21-alkohol in absolutem Dichlormethan versetzt und 18 bis 48 Std. bei 20°C gerührt. Nach üblicher Aufarbeitung kann der gewünschte Ester der Formel I rein dargestellt werden. Anstatt DCCI kann auch Molekularsieb verwendet werden.

In einer weiteren Veresterungsmethode wird 21-Desoxi-corticoid-21-[tert.-Butyldimethylsilyl-(O)-ether] in absolutem Tetrahydrofuran mit 1 Moläqu. Carbonsäure und Trifluoressigsäureanhydrid versetzt, und nach etwa 1 bis 6 Std. Rühren wird bei 20°C wie üblich aufgearbeitet.

Man kann aber auch direkt die Carbonsäure sowie den 17-Desoxi-corticoid-21-alkohol (freie Form) mit Trifluoressigsäureanhydrid zum gewünschten 21-Carbonsäureester umsetzen (= Bildung des gemischten Anhydrids aus Carbonsäure und Trifluoressigsäure, das dann mit dem 21-Alkohol zum 21-Ester reagiert).

### zu Verfahrensvariante b:

Eine weitere vorteilhafte Verfahrensvariante, die zu den erfindungsgemäßen Corticoiden führt, besteht darin, daß man ein(en) 17-Desoxi-corticoid-21-halogenid, vorzugsweise 21-Iodid oder 21-Bromid oder 21-Sulfonat, vorzugsweise 21-p-Chlorbenzolsulfonsäureester oder 21-methansulfonsäureester mit den Metallsalzen, vorzugsweise Alkalisalzen oder Trialkylammoniumsalzen, der in Liste 2 aufgeführten Carbonsäuren in inerten organischen Lösungsmitteln, vorzugsweise Dimethylsulfoxyd, Dimethylformamid, Butanon-(2), Aceton, Acetonitril, 1 bis 16 Std., vorzugsweise 1 bis 10 Std. bei 20°C bis zu den Siedepunkten der verwendeten Lösungsmittel, vorzugsweise ca. 50°C, erhitzt und nach üblicher Aufarbeitung, vorzugsweise Eingießen von Wasser, Abfiltrieren oder Abdekantieren des Niederschlags und üblicher Reindarstellung isoliert.

Für die gemäß Verfahrensweisen a) und b) hergestellten Verbindungen I gilt, daß eine Hydroxygruppe in 11-Stellung gegebenenfalls nach üblichen Methoden zur Ketogruppe oxydiert werden kann. Vorzugsweise wird diese Oxydation mit Chromtrioxid in saurem Medium und in einem inerten organischen Lösungsmittel durchgeführt. Eine im Corticoidteil vorhandene 9(11)-Doppelbindung kann gegebenenfalls durch Addition von Halogenwasserstoffsäure oder durch Chlor nach üblichen bekannten Methoden in die entsprechenden erfindungsgemäßen 17-Desoxi-corticoid-21-ester mit einer 11β-Hydroxyl-9α-Halogenidgruppe (9αF,Cl) oder 11β-9α-Dichlorgruppe überführt werden.

Die Verfahrensprodukte besitzen wertvolle pharmakologische Eigenschaften. Sie sind insbesondere lokal und topisch sehr stark antiphlogistisch wirksam und zeigen teilweise ein überraschend sehr gutes Verhältnis von lokaler zu systemischer antiinflammtorischer Wirkung, das gegenüber strukturverwandten Corticoid-21-estern, die im 21-Esterrest keine Aryl- oder Heteroarylgruppe tragen, so z.B. 21-Estergruppen mit einer 21-Alkylgruppe bzw. gegenüber analogen 17-Desoxi-corticoiden mit nicht veresterter, also freier 21-Hydroxylgruppe, oft deutlich überlegen ist, wie aus pharmakologischen Standardtests hergeleitet werden kann (siehe Pharmakolog. Test-Teil). Demgemäß ist Gegenstand der Erfindung auch ein Mittel zur Behandlung entzündlicher Dermatosen bestehend aus einer Verbindung der Formel I.

Die Verfahrensprodukte können in der Veterinär- und Humantherapie zur Behandlung von entzündlichen Dermatosen verschiedenster Genese in Form von Suspensionen, Salben, Cremes, Sprays usw. Verwendung finden. Dabei ist als besonders vorteilhaft für die lokale und topische Therapieform herauszuheben, daß die Verfahrensprodukte aufgrund ihres äußerst günstigen Verhältnisses von lokaler zu systemischer antiphlogistischer Wirkung auch bei hochdosierter und langanhaltender Therapie praktisch nur geringfügige systemische Nebenwirkungen hervorrufen können. Bei äußerlicher Behandlung werden Salben, Cremes, Suspensionen usw. mit einer Konzentration von 0.01 bis 2 Gew.-% verwendet. Insbesondere zeigen die Verfahrensprodukte in pharmakologischen Tests einen zum Teil wesentlich besseren Split (Verhältnis) von lokaler/systemischer antiinflammatorischer Wirkung als entsprechende Präparate mit einer freien 21-Hydroxylgruppe bzw. mit einer 21-Estergruppe, die im Esterteil keine Aryl- bzw. Heteroarylanteile, wie es bei den erfindungsgemäßen Verbindungen der Fall ist, aufweisen. Weiterhin zeigen die Verfahrensprodukte teilweise auch eine stärkere lokale antiphlogistische Wirksamkeit als die zuletzt genannten Analogpräparate. Darüber hinaus können die erfindungsgemäßen 17-Desoxi-corticoid-21-ester gegenüber den analogen zuletzt genannten Corticoid-derivaten oft eine noch geringere Haut-Atrophogenität aufweisen, was ein weiterer Vorteil für eine dermatotherapeutische Behandlung ist.

17-Desoxi-corticoid-21-zimtsäureester, insbesondere in 4-Stellung im Aromaten durch Methoxy-, Methylendioxy oder Ethoxy substituierte, sowie 17-Desoxi-corticoid-21-[4-(dimethylamino)benzoat] können über ihre antiphlogistische Wirkung hinaus eine zusätzliche Lichtschutzwirkung gegen Sonnen-, insbesondere UV-B und UV-A-Strahlung aufweisen.

Darüber hinaus können die erfindungsgemäßen Verfahrensprodukte in an sich bekannter Weise mit diversen gut hautverträglichen lokal wirksamen Antibiotika, z.B. vom Typ des Gentamycins, Neomycins, Erythromycins, Tetracyclins oder der Fusidinsäure und anderen, in galenischen Formulierungen kombiniert werden. Derartige Kombinationen aus den Verfahrensprodukten und den lokalen wirksamen Antibiotika können zur Behandlung von primären bakteriellen oder bakteriell superinfizierten entzündlichen Dermatosen verwendet werden.

### Pharmakologischer Versuchsteil

So zeigte z.B. Desoximetason-21-zimtsäureester (Verbindung I) eine starke lokale antiphlogistische Wirkung bei einer deutlich verringerten systemischen Wirkung im Vergleich zu Desoximetason, wie aus den unten angeführten Beispielen pharmakologischer Wirksamkeit hervorgeht.

### 1. Lokale antiphlogistische Wirkung im Oxazolon-Ohrödem an Mäusen nach epicutaner Applikation

Es wurde die von Evans, D.P. et al., Br. J. Pharmacol. 43, 403 (1971) beschriebene Methode verwendet. 4-Ethoxymethylen-2-phenyl-2-oxazolin (Oxazolon) verursacht an Mäusen eine allergische Entzündung vom verzögerten Typ, die durch Corticosteroide hemmbar ist. Versuchstiere sind männliche NMRI-Mäuse im Gewicht von 25 g Körpergewicht in Gruppen zu je 10 Tieren. Die Tiere wurden durch Auftragung von 0,1 ml einer 2%igen Lösung von Oxazolon in Aceton auf die geschorene Bauchhaut sensibilisiert. Am Tag 9 nach dieser Sensibilisierung wurde die allergische Entzündung ausgelöst, indem 10 µl einer 2%igen Oxazolon-Aceton-Lösung auf die Innenseite der rechten Ohrmuschel aufgebracht wurden (Kontrollgruppe). Bei den behandelten Gruppen waren die Prüfpräparate in oben genannter Lösung enthalten. 24 Stunden nach der Applikation der Lösungen wurden die Tiere mit CO₂ getötet. Aus der behandelten rechten und der unbehandelten linken Ohrmuschel wurde je eine kreisrunde, 8 mm messende Probe ausgestanzt. Die Proben wurden sofort gewogen, wobei die rechts-links-Differenz das Maß für den Grad der Entzündung darstellt. Diese entzündlich-ödematöse Schwellung in mg wurde bei der Kontrollgruppe = 100 % gesetzt und die entzündungshemmende Wirkung der Präparate ist als prozentuale Hemmung gegenüber der Kontrolle angegeben.

| Behandlung | mg/ml | x ± s | Hemmung in % |
|---|---|---|---|
| Kontrolle | --- | 13,4 ± 3,7 | -- |
| Verb. I | 0,03 | 7,7 ± 3,7 | 43 |
| Verb. I | 0,1 | 3,9 ± 2,9 | 71 |
| Verb. I | 0,3 | 2,3 ± 1,3 | 83 |
| Desoximetason | 0,03 | 8,6 ± 3,1 | 36 |
| Desoximetason | 0,1 | 3,9 ± 3,0 | 71 |
| Desoximetason | 0,3 | 2,8 ± 2,3 | 79 |

Die graphische Auswertung im halblogarithmischen System ergibt in beiden Fällen 50%-Hemmwerte bei 0,05 mg/ml, also Wirkungsgleichheit.

### 2 a. Prüfung auf systemische Wirksamkeit nach subcutaner Gabe im Carrageenin-Pfotenödem an Ratten

Die Methode wurde von Winter, C.A. et al. in Proc. Soc. exp. Biol. (N.Y.), 111, 544 (1962) beschrieben. Männliche Sprague-Dawley-Ratten im Gewicht von ca. 200 g Körpergewicht und mit einer Gruppengröße von n = 5 erhielten die Substanzen s.c. (0,2 ml/100 g Körpergewicht, gelöst in Sesamöl). Kontrollen erhielten nur Sesamöl. 15 Minuten später wurde in die linke Hinterpfote 0,1 ml einer 0,5%igen Carrageenin-Lösung injiziert. Davor und 3 h sowie 6 h später wurde das Pfotenvolumen gemessen (ml) und die Schwellungszunahme gegenüber dem Vorwert ermittelt. Die Zahlen sind Mittelwert und Standardabweichung (x ± s). Die statistische Signifikanzprüfung erfolgte mit dem Test nach Dunnett.

| Behandlung | Dosis [mg/kg] | Vorwert | Zunahme 3 h | Zunahme 6 h |
|---|---|---|---|---|
| Kontrolle | --- | 1,43 ± 0,06 | 0,50 ± 0,05 | 0,46 ± 0,07 |
| Verb. I | 0,1 | 1,49 ± 0,07 | 0,40 ± 0,06 | 0,39 ± 0,12 |
| Desoxi. | 0,1 | 1,46 ± 0,08 | 0,28 ± 0,16* | 0,23 ± 0,09* |
| | | | | |
| Kontrolle | --- | 1,46 ± 0,10 | 0,69 ± 0,16 | 0,53 ± 0,12 |
| Verb. I | 0,3 | 1,51 ± 0,04 | 0,46 ± 0,12 | 0,32 ± 0,05* |
| Desoxi | 0,3 | 1,43 ± 0,07 | 0,17 ± 0,06* | 0,03 ± 0,03* |

Ergebnis: Eine signifikante Wirkung (* = p <0,05) tritt bei Desoximetason bereits mit 0,1 mg/kg ein, nicht jedoch bei Verbindung I. Auch nach 0,3 mg/kg ist Verbindung I noch praktisch wirkungslos, während Desoximetason die Entzündung schon fast vollständig hemmt.

### 2 b. Prüfung auf systemische Wirkung: Glukoneogenese an Ratten

Männliche Sprague-Dawley Ratten im Gewicht von ca. 140 g wurden adrenalektomiert. Sie erhielten 0,9%ige Kochsalzlösung als Trinkwasser. 48 h später wurde den Tieren für 24 h das Futter entzogen. Am Versuchstag (3 Tage nach Adrenalektomie und 1 Tag Hunger) wurden die Versuchspräparate s.c. appliziert (2 ml/kg in Sesamöl, Kontrollen erhielten nur dieses Vehikel). 6 h später wurden die Tiere dekapitiert, und es wurde 1 g Leber entnommen. Die Probe wurde in 5 ml 0,6 M Perchlorsäure aufgenommen. Nach Homogenisierung und Zentrifugation wurde Glukose im Überstand bestimmt. Der Niederschlag (Glycogen) wurde enzymatisch mit Amyloglukosidase aufgeschlossen und darin ebenfalls Glukose bestimmt (Hexokinase-Testkit Boehringer Mannheim). Folgende Ergebnisse wurden erhalten:

| Behandlung | Dosis [mg/kg] | Leberglykogen g/100 g Lebendgewicht | Leberglyk. + Glukose g/100 g Lebendgewicht |
|---|---|---|---|
| Kontrolle | --- | 1,67 ± 0,52 | 14,53 ± 1,95 |
| | | | |
| Verb. I | 0,3 | 1,56 ± 0.50 | 16,91 ± 3,11 |
| Verb. I | 1,0 | 95,86 ± 21,33* | 154,15 ± 4,99* |
| Desoxi. | 0,1 | 38,00 ± 17,43* | 98,55 ± 14,46* |
| Desoxi | 0,3 | 77,90 ± 29,67* | 137,32 ± 28,04* |

Aus dem Ergebnis geht hervor, daß Verbindung I mit 0,3 mg/kg nicht glukoneogenetisch wirkt, während Desoximetason bereits mit 0,1 mg/kg diesen unerwünschten Effekt zeigt (* = p < 0,05, Dunnett's Test). Dieser Effekt tritt bei Verbindung 1 erst mit 1 mg/kg ein, so daß der Vorteil der Verbindung I mit dem Faktor 3 - 10 anzusetzen ist.

Insgesamt ergibt sich aus den Beispielen 1 - 2 b der pharmakologischen Prüfung eine auffallende Minimierung der unerwünschten systemischen Wirkung durch Verbindung I bei Erhalt der lokalen Wirkungsstärke im Vergleich zu Desoximetason.

### Beispiele:

Zu den im folgenden aufgeführten Beispielen sind die nachstehenden allgemeinen Bemerkungen zu machen:
Die Schmelzpunkte werden im Apparat nach Tottoli (Fa. Büchi) oder auf der Kofler-Heizbank der Fa. Reichert (Austria), Typ 7841, bestimmt und sind nicht korrigiert.

Die IR-Spektren (in KBr) werden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckmann DK 1 A. Die massenspektroskopischen Untersuchungen (MS) werden vorwiegend mit dem Gerät MS 9 (Fa. AEI) durchgeführt. Angabe der MS-Spektren (Molgewichtspeak) überwiegend in: MS = m/z = ... (M + H⁺) (Messung mit Reinistopen), d.h. es wurde jeweils die monoisotopische Molmasse erfaßt. In der Regel wurden FAB-MS-Spektren gemessen.
Für die Dünnschicht-Chromatographie (DC) dienten Fertigplatten Kieselgel F₂₅₄ (Fa. Merck). Wenn nicht anders angegeben, wurde als Laufmittel Methylenchorid: Methanol = 19 : 1 benutzt (Laufstrecke 7 cm). Es wurde jeweils zweimal entwickelt. Die Flecken wurden entweder mit einer UV-Lampe bei 254 nm detektiert oder durch Besprühen mit 10 %-iger methanolischer Schwefelsäure sowie durch Erhitzen auf 100°C sichtbar gemacht. Die R_{F}-Werte sind immer nur relativ zu verstehen. Zur Säulenchromatographie wurde 15 Kieselgel 60, Korngröße 0.063 bis 0.2 mm (Fa. Merck) verwendet.

Bei den Reaktionen mit Carbonsäurechloriden wird in vorteilhafter Weise oft absolutes Dioxan zum Reaktionsgemisch gegeben, z.B. bei Benzoylchlorid, wobei das Verhältnis Dioxan/Pyridin etwa 1:1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).

Die Charakterisierung der Reaktionsprodukte kann durch Dünnschicht-Chromatographie (DC) erfolgen; hierbei haben die Reaktionsprodukte R_{F}-Werte von etwa 0.65 bis 0.75. In der Regel werden die Reaktionsprodukte durch Massenspektren mit MS = m/z = ... (M + H⁺) charakterisiert (In der Regel FAB-Spektren); es wird jeweils die monoisotopische Molmasse erfaßt. Die M + H⁺-Werte wurden jeweils aufgerundet. Auch IR-, ¹H-NMR- und UV-Spektren können zur Charakterisierung herangezogen werden.

### Beispiel 1: Desoximetason-21-zimtsäureester

Zu einer Lösung von 6 g Desoximetason in 40 ml absolutem Pyridin wird bei 0°C und unter Rühren eine Lösung von 3,5 g Zimtsäurechlorid in 20 ml absolutem Dioxan zugetropft. Nach 5 Std. Rühren bei 0°C (DC zeigt vollständige Bildung des gewünschten Reaktionsproduktes) gießt man in 1 I halbgesättigte wäßrige Kochsalzlösung ein, isoliert die Ausfällung (Wachs) über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert mit Diethylether oder Diisopropylether oder Petrolether, filtriert ab und kristallisiert gegebenenfalls aus Ethanol/Diethylether (ggf. Zusatz von Diisopropylether oder Petrolether) um. Man erhält 7,5 g der o.a. Titelverbindung vom Schmp. 161°C.
MS: m/z = 507 (M + H⁺)
DC: R_{F} ≅ 0,7

### Beispiel 2: Corticosteron-21-zimtsäureester

In gleicher Weise, wie in Beispiel 1 beschrieben, werden 580 mg Corticosteron in 4 ml absol. Pyridin mit 350 mg Zimtsäurechlorid in 2 ml absol. Dioxan umgesetzt, nach 5 Stunden Rühren bei 0°C aufgearbeitet (Eingießen in 100 ml halbgesättigte Kochsalzlösung usw.) und durch Kristallisieren dargestellt (isoliert). Man erhält 660 mg der o.a. Titelverbindung vom Schmp. 154-157°C.
MS: m/z = 477 (M + H⁺)
DC: R_{F} ≅ 0,7

### Beispiel 3: 11 -Desoxicorticosteron-21-zimtsäureester

In gleicher Weise wie in Beispiel 2 beschrieben, werden 570 mg 11-Desoxicorticosteron anstelle des Corticosterons umgesetzt, dann wird aufgearbeitet und das Produkt isoliert. Man erhält 520 mg der o.a. Titelverbindung vom Schmp. 140 - 143°C.
MS: m/z = 461 (M + H⁺)
DC: R_{F} ≅ 0,75

### Beispiel 4: Fluocortolon-21-zimtsäureester

In gleicher Weise, wie in Beispiel 1 beschrieben, werden 600 mg Fluocortolon in 4 ml absol. Pyridin mit 350 mg Zimtsäurechlorid in 2 ml absol. Dioxan umgesetzt (5 Std. 0°C), es wird aufgearbeitet (Eingießen in 100 ml halbgesättigte Kochsalzlösung usw.) und in kristallisierter Form isoliert.
Man erhält 720 mg der o.a. Titelverbindung vom Schmp. 154-159°C.
MS: m/z = 507 (M + H⁺)
DC: R_{F} ≅ 0,8

### Beispiel 5: Difluorcortolon-21-zimtsäureester

In gleicher Weise, wie in Beispiel 4 beschrieben, werden 610 mg Difluorcortolon anstelle des Fluocortolons umgesetzt; es wird aufgearbeitet und das Produkt isoliert. Aus Diisopropylether (Anreiben) erhält man 560 mg der o.a. Titelverbindung (Schmp. 120-128°C; amorph).
MS: m/z = 525 (M + H⁺)
DC: R_{F} ≅ 0,8

### Beispiel 6: Clocortolon-21-zimtsäureester

In gleicher Weise, wie in Beispiel 4 beschrieben, werden 620 mg Clocortolon anstelle des Fluocortolons umgesetzt; dann wird aufgearbeitet und das Produkt isoliert. Aus Diisopropylether (Anreiben) erhält man 590 mg der o.a. Titelverbindung; amorph.
MS: m/z = 542 (M + H⁺)
DC: R_{F} ≅ 0,8

### Beispiel 7: 9α-Fluor-corticosteron-21-zimtsäureester

In gleicher Weise, wie in Beispiel 4 beschrieben, werden 600 mg 9α-Fluor-corticosteron anstelle des Fluocortolons umgesetzt; es wird aufgearbeitet und das Produkt isoliert. Aus n-Hexan (Anreiben) erhält man 630 mg der o.a. Titelverbindung in amorpher Form.
MS: m/z = 495 (M +H⁺)
DC: R_{F} ≅ 0,8

### Beispiel 8: Desoximetason-21-(4-methoxy-zimtsäure)ester

Zu einer Lösung von 6 g Desoximetason in 40 ml absolutem Pyridin wird bei 0°C und unter Rühren eine Lösung von 4,2 g 4-Methoxy-zimtsäurechlorid in 20 ml absolutem Dioxan zugetropft. Nach 5 Std. Rühren bei 0°C (DC zeigt vollständige Bildung des gewünschten Reaktionsproduktes) gießt man in 1 I halbgesättigte wäßrige Kochsalzlösung ein, isoliert die Ausfällung (Wachs) über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert mit Diethylether oder Diisopropylether oder Petrolether, filtriert ab und kristallisiert gegebenenfalls aus Ethanol/Diethylether (ggf. Zusatz von Diisopropylether oder Petrolether) um. Man erhält 9,4 g der o.a. Titelverbindung vom Schmp. 185°C. Bei einem weiteren Ansatz zeigte das Reaktionsprodukt einen Schmp. von 194°C.
MS: m/z = 537 (M + H⁺)
DC: R_{F} ≅ 0,75

### Beispiel 9: Corticosteron-21-(4-Methoxy-zimtsäure)ester

In gleicher Weise, wie in Beispiel 8 beschrieben, werden 580 mg Corticosteron in 4 ml absol. Pyridin mit 420 mg 4-Methoxy-zimtsäurechlorid in 2 ml absol. Dioxan umgesetzt, nach 5 Stunden Rühren bei 0°C wurde aufgearbeitet (Eingießen in 100 ml halbgesättigte Kochsalzlösung usw.) und das Produkt durch Kristallisieren dargestellt (isoliert). Man erhält 620 mg der o.a. Titelverbindung vom Schmp. ≅ 160°C.
MS: m/z = 507 (M + H⁺)
DC: R_{F} ≅ 0,7

### Beispiel 10: Desoxicorticosteron-21-(4-methoxy-zimtsäure)ester

In gleicher Weise wie in Beispiel 8 beschrieben, werden 570 mg Desoxicorticosteron anstelle des Desoximetasons umgesetzt; es wird aufgearbeitet und das Produkt isoliert. Man erhält 500 mg der o.a. Titelverbindung vom Schmp. 153°C.
MS: m/z = 491 (M + H⁺)
DC: R_{F} ≅ 0,75

### Beispiel 11: Fluocortolon-21-(4-methoxy-zimtsäure)ester

In gleicher Weise, wie in Beispiel 8 beschrieben, werden 600 mg Fluocortolon in 4 ml absol. Pyridin mit 420 mg 4-Methoxy-zimtsäurechlorid in 2 ml absol. Dioxan umgesetzt (5 Std., 0°C), aufgearbeitet (Eingießen in 100 ml halbgesättigte Kochsalzlösung usw.) und in kristallisierter Form isoliert. Man erhält 690 mg der o.a. Titelverbindung vom Schmp. 164-176°C (vorher ab 150°C, Sintern, amorph).
MS: m/z = 537 (M + H⁺)
DC: R_{F} ≅ 0,75

### Beispiel 12: Difluocortolon-21-(4-methoxy-zimtsäure)ester

In gleicher Weise, wie in Beispiel 11 beschrieben, werden 610 mg Difluorcortolon anstelle des Fluocortolons umgesetzt; dann wird aufgearbeitet und das Produkt isoliert. Aus Diisopropylether (Anreiben) erhält man 590 mg der o.a. Titelverbindung (amorph).
MS: m/z = 555 (M + H⁺)
DC: R_{F} ≅ 0,8

### Beispiel 13: Clocortolon-21-(4-methoxy-zimtsäure)ester

In gleicher Weise, wie in Beispiel 11 beschrieben, werden 620 mg Clocortolon anstelle des Fluocortolons umgesetzt; dann wird aufgearbeitet und das Produkt isoliert. Aus Diisopropylether (Anreiben) erhält man 620 mg der o.a. Titelverbindung; amorph.
MS: m/z = 572 (M + H⁺)
DC: R_{F} ≅ 0,8

### Beispiel 14: 9α-Fluor-corticosteron-21-(4-methoxy-zimtsäure)ester

In gleicher Weise, wie in Beispiel 11 beschrieben, werden 600 mg 9α-Fluor-corticosteron anstelle des Fluocortolons umgesetzt; dann wird aufgearbeitet und das Produkt isoliert. Aus Diisopropylether (Anreiben) erhält man 680 mg der o.a. Titelverbindung (amorph).
MS: m/z = 525 (M + H⁺)
DC: R_{F} ≅ 0,8

### Beispiel 15: Desoximetason-21-(4-phenyl)zimtsäureester

Zu einer Lösung von 3,0 g Desoximetason und 2,3 g 4-Phenylzimtsäure in 60 ml absol. Methylenchlorid gibt man bei 0°C und Rühren 96 mg 4-Dimethylaminopyridin und 2,0 g Dicyclohexylcarbodiimid. Die zunächst klare Reaktionslösung trübt sich bald. Nach ca. 6 Stunden Rühren bei Zimmertemperatur zeigt eine DC-Probe kein Edukt mehr. Man bewahrt dann 2 Tage bei +4°C und 2 Tage bei -15°C (Tiefkühlschrank) auf, filtriert den ausgefallenen Dicyclohexylharnstoff ab, wäscht diesen mit etwas -15°C kaltem Methylenchlorid und zieht das organische Lösungsmittel im Vakuum ab. Der hinterbliebene Rückstand wird aus siedendem Diethylether zur Kristallisation gebracht und gegebenenfalls aus Ethanol/Diethylether umkristallisiert. Man erhält 4,1 g der o.a. Titelverbindung vom Schmp. 142°C.
MS: m/z = 583 (583,3)-(M + H⁺)
DC: R_{F} ≅ 0,75

### Beispiel 16: Desoximetason-21-(trans-3,4-methylendioxy)zimtsäureester

In gleicher Weise, wie in Beispiel 15 beschrieben, werden 3 g Desoximetason mit 2,0 g trans-3,4-Methylendioxy-zimtsäure anstelle von 4-Phenylzimtsäure umgesetzt, aufgearbeitet, isoliert und rein dargestellt. Man erhält 1,9 g der o.a. Titelverbindung; Schmp. 147 - 151°C.
MS: m/z = 551 (M + H⁺)
DC: R_{F} ≅ 0,7

### Beispiel 17: Desoximetason-21-(trans-3,4-dimethoxy)zimtsäureester

In gleicher Weise, wie in Beispiel 15 beschrieben, werden 3 g Desoximetason mit 2,0 g trans-3,4-Dimethoxy-zimtsäure anstelle von 4-Phenylzimtsäure umgesetzt, aufgearbeitet, isoliert und rein dargestellt. Man erhält 2,4 g der o. a. Titelverbindung mit Schmp. 139 - 144°C.
MS: m/z = 567 (M + H⁺)
DC: R_{F} = 0,75

Werden in Beispiel 17 anstelle von 2,0 g trans-3,4-Dimethoxy-zimtsäure eine äquivalente Menge von trans-2,3-Dimethoxy-zimtsäure oder trans-2,4-Dimethoxy-zimtsäure oder trans-2,5-Dimethoxy-zimtsäure oder trans-3,5-Dimethoxy-zimtsäure in die Reaktion eingesetzt, so erhält man nach analoger Reaktionsführung, Aufarbeitung und Isolierung jeweils die entsprechenden Desoximetason-21-trans-2,3-(oder 2,4-, oder 2,5-, oder 3,5-)dimethoxy-zimtsäureester, alle mit MS: m/z = 567 (M + H⁺).

### Beispiel 18 (p steht für 4): Desoximetason-21-(p-methyl-zimtsäure)ester

In gleicher Weise, wie in Beispiel 15 beschrieben, werden 3 g Desoximetason mit 1,9 g p-Methylzimtsäure anstelle von 4-Phenylzimtsäure umgesetzt, dann wird aufgearbeitet, isoliert und rein dargestellt. Man erhält 2,1 g der o. a. Titelverbindung; Schmp. 171°C.
MS: m/z = 521 (M + H⁺)
DC: R_{F} ≅ 0,7

### Beispiel 19

Werden in Beispiel 18 anstelle von p- bzw. 4-Methylzimtsäure 1,9 g α-Methylzimtsäure (= C₆H₅CH =C(CH₃)CO₂H) in die Reaktion eingesetzt, so erhält man nach analoger Reaktionsführung, Aufarbeitung und Isolierung den isomeren Desoximetason-21-(α-methylzimtsäure)ester (amorphes Kristallisat nach Fällung mit Diethylether).
MS: m/z = 521 (M + M⁺)
DC: R_{F} ≅ 0,75

Wird in Beispiel 18 anstelle von α- eine entsprechende Menge (1,9 g) β-Methylzimtsäure (z. B. trans-) in die Reaktion eingesetzt, so erhält man den Desoximetason-21-(β-methyl-zimtsäure)ester.

### Beispiel 20: Desoximetason-21-phenylpropiolsäureester

In gleicher Weise, wie in Beispiel 15 beschrieben, werden 3 g Desoximetason mit 1,9 g Phenylpropiolsäure anstelle von 4-Phenylzimtsäure umgesetzt (Reaktionszeit 24 Std.); es wird aufgearbeitet und das Produkt isoliert. Aus dem resultierenden dunklen Öl (2,2 g) kristallisiert nach mehreren Tagen die o.a. Titelverbindung in kristallinierter Form langsam aus, die nur schlecht rein dargestellt werden kann. Ausmessung des ölig-kristallinen Rohprodukts:
MS: m/z = 505 (M + H⁺)
DC: R_{F} ≅ 0,8

### Beispiel 21: Desoximetason-21-(5-phenyl-penta-2,4-diensäure)ester

In gleicher Weise, wie in Beispiel 15 beschrieben, werden 3 g Desoximetason mit 1,6 g 5-Phenyl-penta-2,4-diensäure (= Cinnamylideneacetic acid) anstelle von 4-Phenylzimtsäure umgesetzt, dann wird aufgearbeitet, die Substanz isoliert und rein dargestellt. Man erhält 3,1 g der o.a. Titelverbindung mit Schmp. 140-146°C (unscharf).
MS: m/z = 533 (M + H⁺)
DC: R_{F} ≅ 0,75

### Beispiel 22: Desoximetason-21-[-3-(3-Furyl)acrylsäureester]

Zu einer Lösung von 500 mg Desoximetason (1,3 mMol) in 3 ml absolutem Pyridin wird bei 0°C und unter Rühren eine Lösung von 254 mg 3-Furylacrylsäurechlorid (1,6 mMol) in 2 ml absolutem Dioxan zugetropft. Nach 4 Stunden Rühren bei 0°C und 62 Stunden Stehen (= übers Wochenende) im Kühlschrank bei +4°C (DC zeigt vollständige Bildung des gewünschten Reaktionsproduktes; R_{F} ≅ 0,8 (Desoximetason hat 0,6)) filtriert man bei +4°C vom ausgefallenen Niederschlag ( = Pyridiniumhydrochlorid) ab. Die Lösungsmittel des erhaltenen Filtrats werden im Hochvakuum weitgehend abdestilliert. Der erhaltene Rückstand wird mit Diethylether angerieben, und das erhaltene Kristallisat wird abfiltriert und mit Diethylether mehrfach gewaschen. Falls gewünscht, kann das erhaltene Kristallisat aus Ethanol/Diethylether (ggf. unter Zusatz von Dichlormethan zur vollständigen Auflösung) umkristallisiert werden. Man erhält 580 mg der o. a. Titelverbindung vom Schmp. 216°C.
MS: m/z = 497 (M + H⁺)
DC: R_{F} ≅ 0,8

Zur Synthese der isomeren Verbindungen Desoximetason-21-[3-(2-thienyl)acrylsäureester] bzw. Desoximetason-21-[3-(2-furyl)acrylsäureester] geht man zweckmäßigerweise von den freien Säurereagenzien 2-Thienylacrylsäure bzw. 2-Furylacrylsäure (anstelle von 4-Phenylzimtsäure) aus und führt die Reaktion gemäß Beispiel 15 durch.

### Beispiel 23: Desoximetason-21-[3-(3-thienyl)acrylsäureester)

In gleicher Weise, wie in Beispiel 22 beschrieben, werden 0,5 g Desoximetason mit 2,75 mg 3-Thienylacrylsäurechlorid anstelle des dortigen Säurechlorids umgesetzt; es wird analog aufgearbeitet, und das Produkt wird kristallisiert rein dargestellt. Aus Diethylether erhält man 580 mg der o. a. Titelverbindung.
Schmp.: 219°C
MS: m/z = 513 (M + H⁺); DC: R_{F} ≅ 0,8

Analog zu diesen Beispielen sind die folgenden Beispiele von Tabellen 1 und 2, wobei R(1)' die gesamte Seitenkette an der 21CH₂O-Gruppe darstellt.

Zur Charakterisierung der Syntheseprodukte wurden jeweils lediglich die nach den Massenspektren erhaltenen Molgewichtspeaks (m/z = .... (M + H⁺)) ausgewertet (als Öl oder Wachs oder amorph oder kristallisiert), und in der Regel erfolgte hiernach keine Reinstdarstellung durch Kristallisation (Umkristallisation) bzw. Chromatographie.

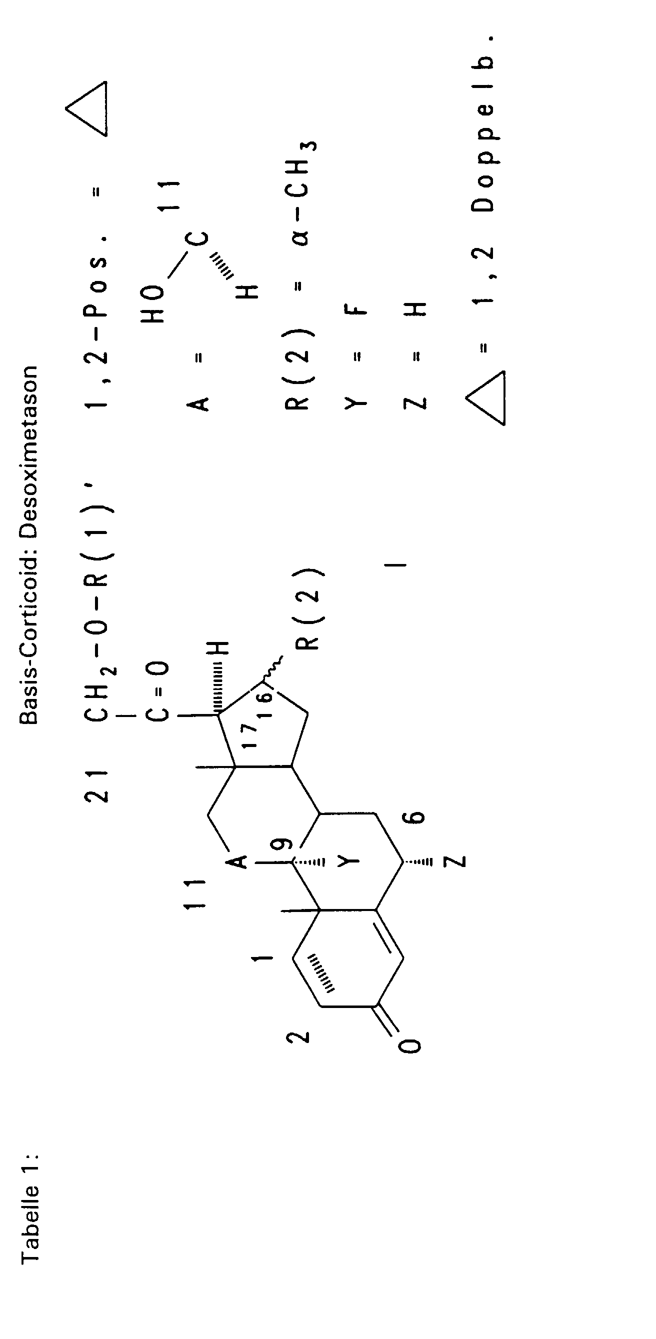

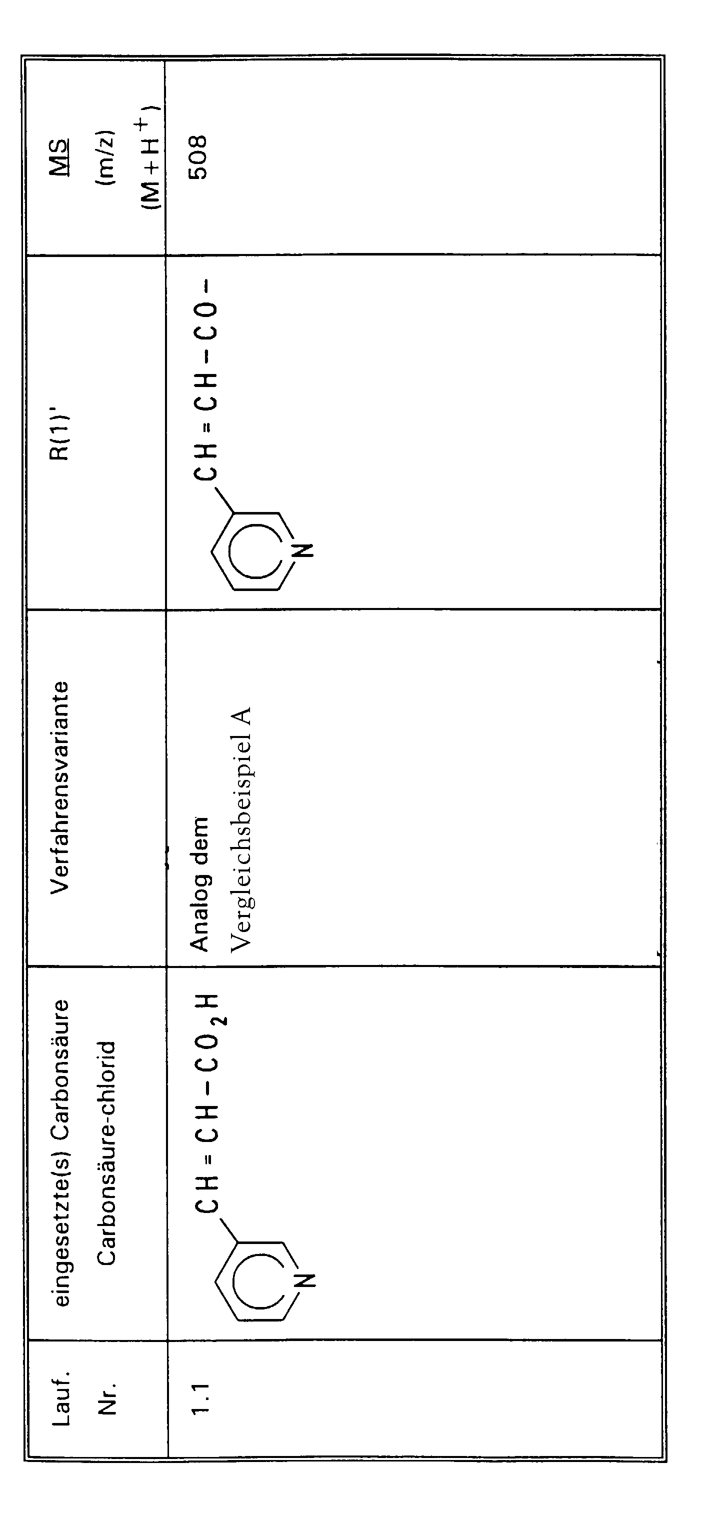

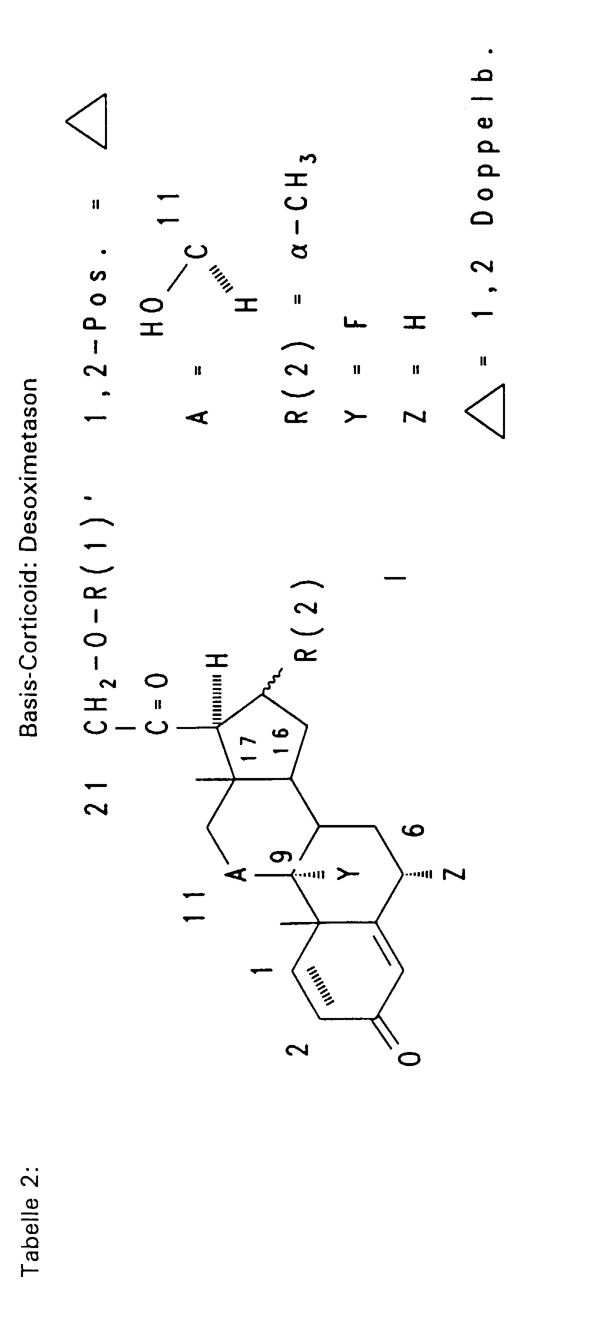

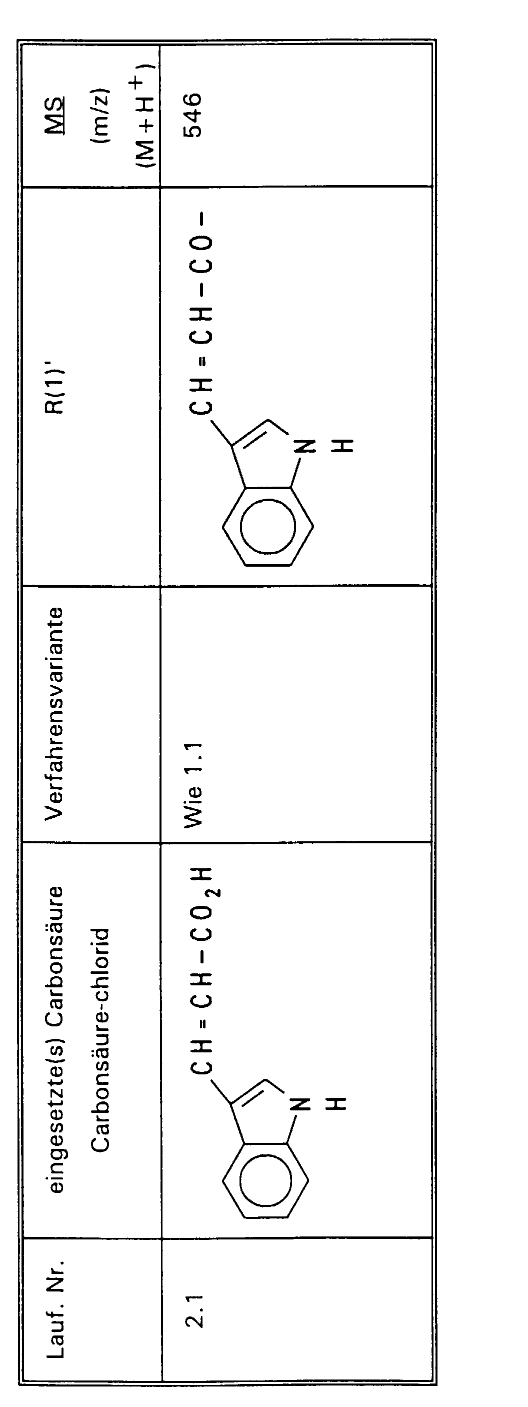

### Liste 2

A) Folgende Carbonsäuren der Formel III [R(5) = OH] bzw. deren aktivierte Derivate, kommen als Ausgangssubstanzen beispielsweise in Frage (die Aryl-bzw. Heteroarylgruppen darin entsprechen den Substituenten R(1):
cis- oder (bevorzugt) trans-Zimtsäure; 2-, 3- oder 4-Methoxy-zimtsäure; 4-Ethoxy-zimtsäure; 3,4-Dimethoxyzimtsäure; 3,4,5-Trimethoxyzimtsäure; 4-Fluor-zimtsäure; 3- oder 4-Chlor-zimtsäure; 3-Brom-zimtsäure; 2- oder 3-Nitro-zimtsäure; 4-Cyan-zimtsäure; 4-Isopropyl-zimtsäure; 4-(t-Butyl)-zimtsäure, 2- oder 4-Trifluormethyl-zimtsäure;
Phenylpropiolsäure; 2-Methyl-3-(4-tetradecyloxyphenyl)-2-propenoic acid (MTPA);
3-(Phenylmercapto)acrylsäure;
3-(2- oder 3-Furyl)acrylsäure; 3-(2-Thienyl)acrylsäure; 3-(3-Thienyl)acrylsäure; 3-(4- oder 2-Pyridyl)acrylsäure; 3-(4-Imidazolyl)acrylsäure;

b.)Annelierte Säuren
3,4-(Methylendioxy)-zimtsäure;

### Vergleichsbeispiel A

### Desoximetason-21-(indol-3-essigsäure)ester

Zu einer Lösung von 1,92 g Desoximetason und 3,1 g 3-Indolessigsäure (getrocknet) in 15 ml absolutem Pyridin gibt man unter Rühren und bei 20° Pyridiniumsulfat (aus 56 mg konz. Schwefelsäure in 2,5 ml absol. Pyridin, gemäß Beispiel 2 b). Nach 30 Minuten Rühren (20°C) gibt man 1,55 g N,N'-Dicyclohexylcarbodiimid hinzu. Nach 48 Stunden Rühren bei 20°C zeigt das Massenspektrum m/z = 534,2 (M + H⁺) und kein m/z = 377 (M + H⁺) für das Ausgangssteroid. Nach analoger Weiterbehandlung und Aufarbeitung wie unter Beispiel 3 b) erhält man nach dem Eingießen in ca. 500 ml halbgesättigte Kochsalzlösung eine ölige Ausfällung, die in ein Wachs übergeht. Man dekantiert bzw. filtriert das Wachs ab, wäscht es mit Wasser und trocknet es im Exsikkator im Vakuum über P₂O₅. Nach dem Anreiben mit Petrolether erhält man 1,35 g der Titelverbindung als amorphes Produkt.
MS (von Wachs bzw. amorphen Material): m/z = 534 (M + H⁺)
DC ≅ 0,75 (Hauptfleck = Hf + wenige schwache Nebenflecke). Zur Reinstdarstellung wird mit Methylenchlorid/Methanol = 99,5:0,5 an Kieselgel chromatographiert (Säule: Durchmesser = 5 cm; h = 20 cm). Die mit R_{F} ≅ 0,75 anfallenden Eluat-Fraktionen werden vereinigt und von den Lösungsmitteln durch Destillation befreit. Der Rückstand wird aus Diethylether zur Kristallisation gebracht. Man erhält 1,0 g der Titelverbindung vom Schmp. ~ 160°C mit den gleichen Daten für MS und DC wie die wachsartige bzw. amorphe Titelverbindung.
MS: m/z = 534 (M + H⁺)
DC: R_{F} ≅ 0,75

## Patentansprüche

1. Ein 17-Desoxi-corticoid-21-carbonsäureester der Formel I 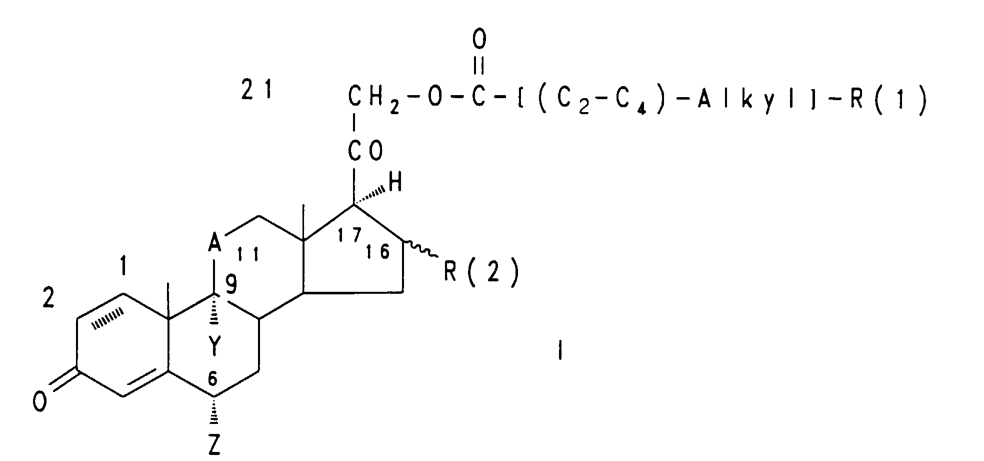 in welcher bedeuten:
A CHOH und CHCI in beliebiger sterischer Anordnung, CH₂, oder C=O;
Y Wasserstoff, Fluor oder Chlor;
Z Wasserstoff, Fluor oder Methyl;
R(1) ggf. substituiertes oder annelliertes Aryl, Heteroaryl;
[(C₂-C₄)-Alkyl] ungesättigt, ab C₃ auch mehrfach ungesättigt;
die Positionen 1,2 gesättigt oder ungesättigt sind,
R(2) Wasserstoff, α- oder β-Methyl.

2. Ein 17-Desoxi-corticoid-21-carbonsäureester I nach Anspruch 1, dadurch gekennzeichnet, daß darin bedeuten:
R(1) wie in Anspruch 1 definiert;
A CHOH, das β-konfiguriert ist;
Y F;
Z Wasserstoff;
R(2) α-Methyl.

3. Verfahren zum Herstellen einer Verbindung I nach Anspruch 1, dadurch gekennzeichnet, daß man
a) eine Verbindung der Formel II, 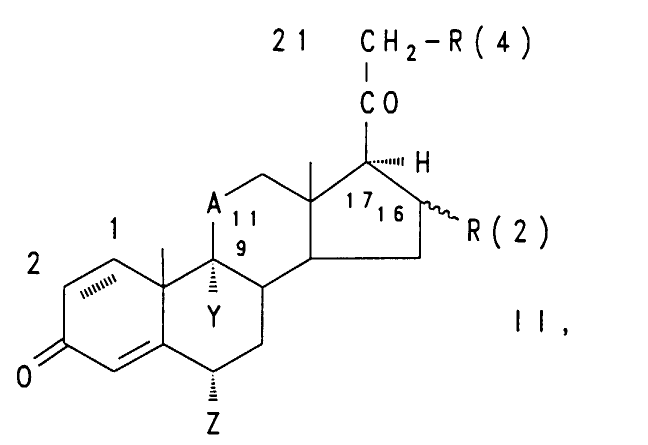 in der R(4) gleich OH ist und die übrigen Substituenten die oben angegebenen Bedeutungen haben,
a 1) mit einer aktivierten Carbonsäure der Formel III, vorzugsweise einem Halogenid oder Anhydrid oder Azolid,
R(5)-CO-[(C₂-C₄)-Alkyl]-R(1) III
umsetzt, wobei
[(C₂-C₄)-Alkyl] und R(1) die in Anspruch 1 angegebenen Bedeutungen haben und
R(5) Cl, Br, O[-CO-[(C₂-C₄)-Alkyl]-R(1)]₁-, -OC(O)CF₃ oder ein anderes aktiviertes Säureradikal, oder
a 2) mit einer Carbonsäure der Formel III selbst, in der
R(5) OH
und die weiteren Substituenten bei Formel III angegeben sind,
in Gegenwart Wasser abspaltender Reagentien umsetzt
oder daß man
b) Verbindungen der Formel II, 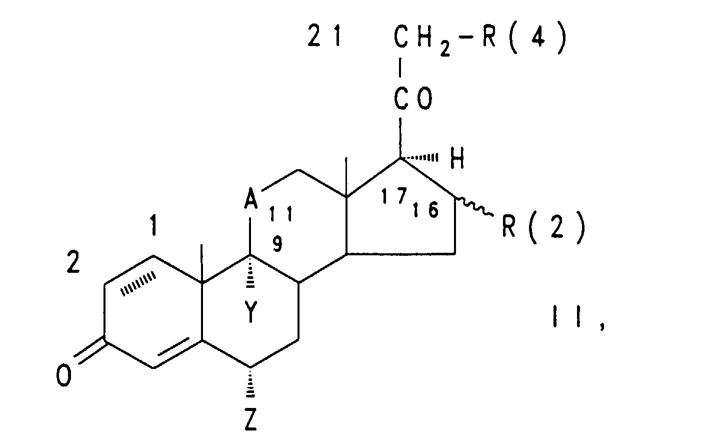 in der R(4) = Br, J, eine Sulfonsäurearyl- oder -alkylestergruppierung ist und die weiteren Substituenten die bei Formel I angegebenen Bedeutung haben, mit einem Salz, vorzugsweise K- oder Na-Salz oder einem Trialkylammoniumsalz, einer Carbonsäure der Formel III,
R(5)-CO-[(C₂-C₄)-Alkyl]-R(1) III
in der
R(5) -[O⁻Me⁺]
und die weiteren Substituenten die bei Formel III angegebenen Bedeutungen haben,
umsetzt,
wobei Me⁺ vorzugsweise das Kation eines Alkalisalzes oder eines Trialkylammoniumsalzes ist.

4. Medikament, gekennzeichnet durch einen wirksamen Gehalt einer Verbindung I nach Anspruch 1.

5. Verwendung einer Verbindung I nach Anspruch 1 zum Herstellen eines Medikaments zur Behandlung von Dermatosen.

## Claims

1. A 17-deoxycorticoid-21-carboxylic ester of the formula I 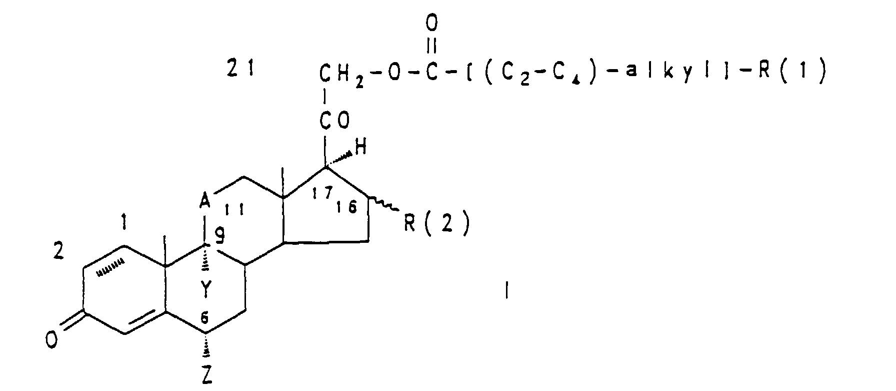 in which:
A is CHOH and CHCl in arbitrary steric arrangement, CH₂ or C=O;
Y is hydrogen, fluorine or chlorine;
Z is hydrogen, fluorine or methyl;
R(1) is optionally substituted or fused aryl or heteroaryl;
[(C₂-C₄)-alkyl] is unsaturated, also unsaturated more than once from C₃ onwards;
the 1,2 positions are saturated or unsaturated,
R(2) is hydrogen or α-methyl or β-methyl.

2. A 17-deoxycorticoid-21-carboxylic ester I as claimed in claim 1 in which:
R(1) is defined as in claim 1;
A is CHOH which is in the β configuration;
Y is F;
Z is hydrogen;
R(2) is α-methyl.

3. A process for preparing a compound I as claimed in claim 1, wherein
a) a compound of the formula II 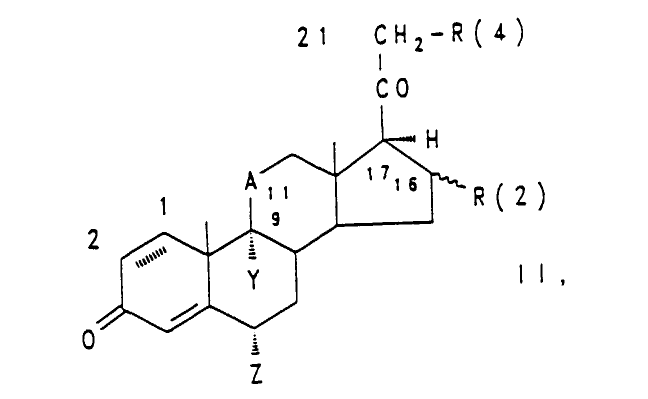 in which R(4) is OH and the remaining substituents have the abovementioned meanings,
a1) is reacted with an activated carboxylic acid of the formula III, preferably a halide or anhydride or azolide,
R(5)-CO-[(C₂-C₄)-alkyl]-R(1) III
in which
[(C₂-C₄)-alkyl] and R(1) have the meanings given in claim 1, and
R(5) is Cl, Br, O[-CO-[(C₂-C₄)-alkyl]-R(1)]₁-, -OC-(O)CF₃, or another activated acid radical, or
a2) is reacted with a carboxylic acid of the formula III itself, in which
R(5) is OH
and the other substituents are given in formula III,
in the presence of water-eliminating reagents,
or wherein
b) compounds of the formula II 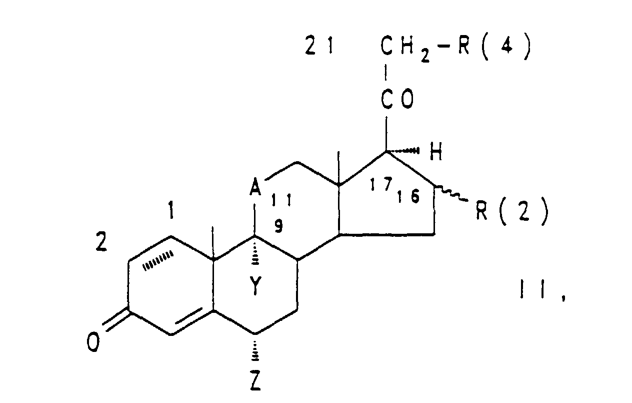 in which R(4) is Br, I, or a sulfonic aryl ester group or sulfonic alkyl ester group, and the other substituents have the meaning given in formula I, are reacted with a salt, preferably a K or Na salt or a trialkylammonium salt, of a carboxylic acid of the formula III,
R(5)-CO-[(C₂-C₄)-alkyl]-R(1) III
in which
R(5) is - [O⁻Me⁺]
and the other substituents have the meanings given in formula III,
Me⁺ preferably being the cation of an alkali metal salt or of a trialkylammonium salt.

4. A pharmaceutical which has an effective content of a compound I as claimed in claim 1.

5. The use of a compound I as claimed in claim 1 for preparing a pharmaceutical for treating dermatoses.

## Revendications

1. 21-ester de 17-désoxycorticoïde avec un acide carboxylique, de formule I 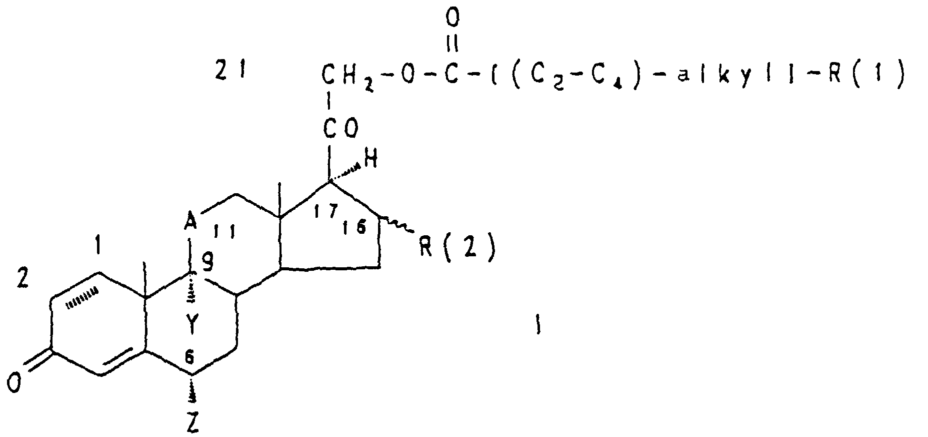 dans laquelle
A représente CHOH ou CHCl en configuration stérique quelconque, CH₂ ou C=O;
Y représente un atome d'hydrogène, de fluor ou de chlore;
Z représente un atome d'hydrogène ou de fluor ou le groupe méthyle;
R(1) représente un groupe aryle, hétéroaryle, éventuellement substitué ou condensé,
[(C₂-C₄)-alkyl] insaturé, à partir de C₃ également plusieurs fois insaturé;
les positions 1,2 sont saturées ou insaturées,
R(2) représente un atome d'hydrogène ou un groupe α- ou β-méthyle.

2. 21-ester de 17-désoxycorticoïde avec un acide carboxylique I selon la revendication 1, caractérisé en ce que, dans celui-ci:
R(1) est défini comme dans la revendication 1;
A représente le groupe CHOH qui est en configuration β;
Y est F;
Z est un atome d'hydrogène;
R(2) représente le groupe α-méthyle.

3. Procédé pour la préparation d'un composé I selon la revendication 1, caractérisé en ce que
a) on fait réagir un composé de formule II 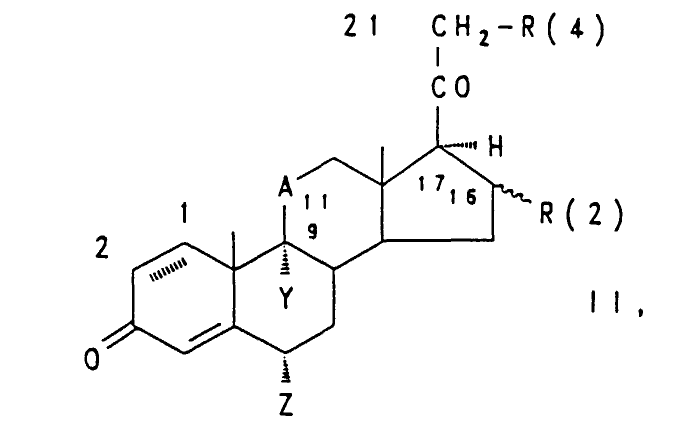 dans laquelle R(4) représente OH et les autres substituants ont les significations données plus haut,
a1) avec un acide carboxylique activé de formule III, de préférence un halogénure ou anhydride ou azolide,
R(5)-CO-[alkyl(C₂-C₄)]-R(1) III
formule dans laquelle
le groupe alkyle en C₂-C₄ et R(1) ont les significations données dans la revendication 1 et
R(5) représente Cl, Br, un groupe O([-CO-[alkyl(C₂-C₄)]-R(1)]₁-, -OC(O)CF₃ ou un autre reste d'acide activé, ou
a2) avec un acide carboxylique de formule III lui-même, dans lequel R(5) représente OH,
et les autres substituants sont indiqués à propos de la formule III
en présence de réactifs éliminant l'eau,
ou en ce que
b) on fait réagir des composés de formule II 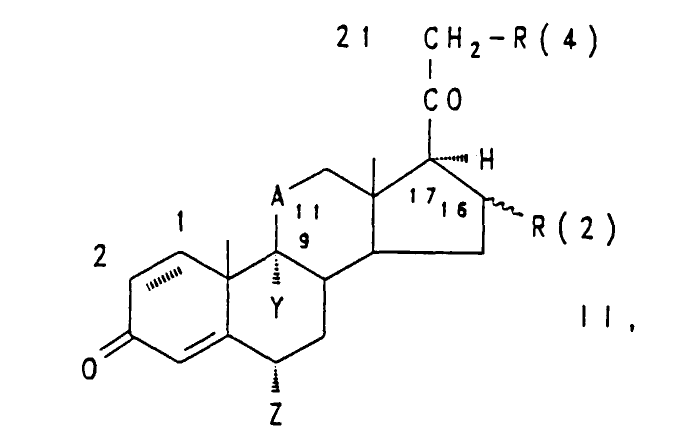 dans laquelle R(4) est Br, I ou un groupement sulfonate d'aryle ou d'alkyle et les autres substituants ont les significations données à propos de la formule I,
avec un sel, de préférence un sel de K ou Na, ou un sel de trialkylammonium, d'un acide carboxylique de formule III
R(5)-CO-[alkyl(C₂-C₄)]-R(1) III
dans laquelle R(5) représente -[O⁻Me⁺] et les autres substituants ont les significations indiquées à propos de la formule III,
Me⁺ étant de préférence le cation d'un sel alcalin ou d'un sel de trialkylammonium.

4. Médicament, caractérisé par une teneur efficace en un composé I selon la revendication 1.

5. Utilisation d'un composé I selon la revendication 1, pour la fabrication d'un médicament destiné au traite de dermatoses.
